# EUROPÄISCHE PATENTSCHRIFT

(11) **EP 0 660 730 B1**
(45) Veröffentlichungstag und Bekanntmachung des Hinweises auf die Patenterteilung: **01.05.1996**
(21) Anmeldenummer: 93918991.6
(22) Anmeldetag: 10.09.1993
(51) Int. Cl.: A61M 5/44

(54) **VORRICHTUNG ZUR HANDHABUNG VON IN DEFORMIERBAREN BEHÄLTNISSEN GESPEICHERTEN STOFFEN**
DEVICE FOR HANDLING MATERIALS STORED IN FLEXIBLE CONTAINERS
DISPOSITIF POUR LA MANIPULATION DE SUBSTANCES STOCKEES DANS DES RECIPIENTS DEFORMABLES

(30) Priorität: 15.09.1992 DE 4230774
(43) Veröffentlichungstag der Anmeldung: 05.07.1995
(73) Patentinhaber: WWT TECHNISCHER GERÄTEBAU GmbH, D-70190 Stuttgart (DE)
(72) Erfinder: THEILACKER-BECK, Wolfgang, D-70597 Stuttgart (DE); STIHLER, Axel, D-70839 Gerlingen (DE); WEEGE, Jens-Peter, D-70199 Stuttgart (DE)
(74) Vertreter: KOHLER SCHMID + PARTNER
(86) Internationale Anmeldenummer: DE9300854
(87) Internationale Veröffentlichungsnummer: WO9406496

(56) Entgegenhaltungen:
- WO-A-88/07384
- WO-A-91/10615
- DE-U- 8 606 622
- DE-U- 9 110 742
- US-A- 4 857 055

## Beschreibung

Die Erfindung betrifft eine medizinische Auftauvorrichtung zum Auftauen von in deformierbaren Behältnissen gespeicherten medizinischen Produkten, insbesondere Blutplasma, Infusionen, Transfusionen oder dergleichen mit einem Gehäuse, in dem das Behältnis angeordnet ist, wobei das im Behältnis gespeicherte Produkt über eine Flüssigkeit einer Druckbehandlung aussetzbar ist, an das Behältnis eine Leitung angekoppelt ist, über die der im Behältnis gespeicherte, flüssige Produktanteil herausgepreßt wird, die medizinische Auftauvorrichtung eine erste Gehäuseteilhälfte und eine zweite Gehäuseteilhälfte aufweist, die zusammenfügbar sind, und wobei das Behältnis innerhalb eines von den Gehäuseteilhälften umschlossenen Freiraumes angeordnet ist.

Eine derartige medizinische Auftauvorrichtung ist durch die WO 88/07384 bekanntgeworden.

Im medizinischen Anwendungsbereich ist es notwendig, Plasmalösungen, Blutkonserven oder andere in der Anwendung flüssige Substanzen gekühlt oder tiefgefroren zu lagern. Diese Produkte müssen im Bedarfsfall schnell verfügbar sein. Das heißt, sie müssen in kürzester Zeit aufgetaut und auf eine vorbestimmte Temperatur schnell erwärmt werden können, damit die Substanzen dem Patienten in flüssiger Form und mit vorbestimmter Temperatur appliziert werden können.

Bekannterweise werden tiefgefrorene medizinische Produkte in Mikrowellengeräten oder Wasserbädern aufgetaut. Dabei wird im wesentlichen darauf geachtet, daß diese Produkte den Aggregatzustand ändern, d. h., daß sie in möglichst kurzer Zeit in flüssiger Form vorliegen.

Sind die zu verabreichenden Flüssigkeiten derart temperiert` daß sie dem Patienten über ein geeignetes Verbindungssystem beispielsweise intravenös gefahrlos zugeführt werden können, so werden diese Flüssigkeiten z. B. tropfenweise verabreicht. Das sehr unterschiedliche Viskositätsverhalten von medizinischen Flüssigkeiten erschwert vielfach die Abgabe der Flüssigkeit unter der reinen Nutzung der Schwerkraft oder einem natürlichen Druckgefälle. Sollen medizinische Flüssigkeiten schnell verabreicht werden, so sind Druckmanschetten bekannt, über die die in Kunststoffbeuteln gespeicherten Infusionslösungen aus den Beuteln herausgepreßt werden können. Vielfach muß jedoch einer aus einer Druckmanschette strömenden Flüssigkeit noch ein Erwärmungsgerät für medizinische Lösungen nachgeschaltet werden, damit beispielsweise eine körpergerechte Temperatur der zu applizierenden Flüssigkeit schnellstmöglich erreicht wird.

Aus der WO 88/07384 ist eine medizinische Auftauvorrichtung bekannt, bei der ein mit Blutplasma gefüllter Plastikbeutel in ein zweiteiliges Gehäuse gehängt wird, bei dem eine Gehäusehälfte mit einer Heizung versehen ist und die andere Gehäusehälfte ein Druckkissen aufweist. Wenn das Gehäuse geschlossen ist, kann in dem Druckkissen hydraulisch oder pneumatisch ein Überdruck aufgebaut werden, der auf den Blutbeutel drückt. Durch die in der Gehäusewand angeordnete Heizvorrichtung wird der Blutbeutel erwärmt und das Blutplasma aufgetaut. Durch den am Blutbeutel anliegenden Druck wird das bereits flüssige Blut über eine Abflußleitung aus dem Blutbeutel herausgedrückt.

Hierbei tritt die Schwierigkeit auf, daß die Wärmezufuhr nur auf einer Seite des Blutbeutels erfolgt, so daß zum Auftauen des Blutes eine sehr große Zeitspanne benötigt wird, insbesondere auch deshalb, weil die maximale Heiztemperatur 41° C aus medizinischen Gründen nicht überschritten werden darf. Eine weitere Schwierigkeit besteht darin, daß der Druckbeutel nur auf eine Seite des Blutbeutels drückt und somit ein vollständiges Entleeren des Blutbeutels nicht erfolgen kann.

Aufgabe der vorliegenden Erfindung ist es, die vorbekannten Einrichtungen derart weiterzubilden, daß in einem Arbeitsgang sowohl die Erwärmung von in deformierbaren Behältnissen gespeicherten medizinischen Produkten als auch deren Dosierung möglich ist, und daß das medizinische Produkt schneller erwärmt wird und der Behältnisinhalt vollständig genutzt wird.

Diese Aufgabe wird erfindungsgemäß dadurch gelöst, daß Heizmittel für die der Druckbehandlung dienende Flüssigkeit vorgesehen sind derart, das im Behältnis gespeicherte Produkt über die Flüssigkeit einer Wärmebehandlung aussetzbar ist, daß an den Gehäusehälften bewegbare Folien angeordnet sind, die die Gehäusehälften abschließen, daß der Freiraum für die Aufnahme des Behältnisses zwischen den Folien der Gehäuseteilhälften bei zusammengefügten Gehäuseteilhälften ausgebildet ist, wobei sich die Folien eng an das Behältnis anlegen, und daß an den Gehäuseteilhälften Anschlüsse für die Führung der Flüssigkeit vorgesehen ist derart, daß das Behältnis vollkommen von der Flüssigkeit indirekt umströmt wird.

Die erfindungsgemäße medizinische Auftauvorrichtung hat damit den wesentlichen Vorteil, daß z. B. ein tiefgefrorener Beutel in einer Vorrichtung aufgetaut, erwärmt und dosiert entleert werden kann. Dabei wird der Beutel vollständig entleert, da der Druck der deformierbaren Beutel von allen Seiten her zusammendrückt.

Der tiefgefrorene Beutel wird in die erfindungsgemäße medizinische Auftauvorrichtung eingelegt und die Flüssigkeit umströmt indirekt das Behältnis in dem Gehäuse vollkommen, so daß eine Erwärmung der im Beutel gespeicherten Substanz bestmöglich stattfinden kann. Werden die Substanzen unmittelbar, sobald sie den Aggregatzustand gewechselt haben, aus dem Beutel herausgepreßt, so ist gewährleistet, daß immer ein sehr großes Temperaturgefälle zwischen der erwärmten Flüssigkeit und der noch festen, im Behältnis gespeicherten Substanz besteht. Die Wärmeübertragung ist in der erfindungsgemäßen medizinische Auftauvorrichtung gegenüber bekannten Geräten erheblich verbessert.

Die erfindungsgemäße medizinische Auftauvorrichtung weist eine erste Gehäuseteilhälfte und eine zweite Gehäuseteilhälfte auf, die zusammenfügbar sind. Die zusammengefügten Gehäuseteilhälften weisen im Inneren einen Freiraum auf, in dem das zu erwärmende und leerzupressende Behältnis angeordnet werden kann. In die Gehäuseteilhälften kann eine erwärmte oder eine unter Druck stehende Flüssigkeit einströmen. Die erwärmte und unter Druck stehende Flüssigkeit umströmt indirekt das Behältnis vollkommen, so daß eine schnelle Erwärmung der im Behältnis gespeicherten Substanz möglich ist. Die erwärmte und/oder verflüssigte Substanz kann über die aus dem Gehäuse herausführende Leitung ausgepreßt werden, so daß im Beutel immer nur die feste Substanz (z. B. gefrorenes Blutplasma) erwärmt wird (falls dies vom Anwender gewünscht wird). In den Gehäuseteilhälften sind Stutzen und Anschlüsse vorgesehen, über die die erwärmte und unter Druck stehende Flüssigkeit strömungsgünstig in die Gehäuseteilhälften ein- und ausströmen kann. Sind die Gehäuseteilhälften über Scharniere miteinander verschwenkbar gekoppelt, so sind Verbindungsschläuche an den Gehäuseteilhälften angeordnet, die die Gehäuseteilhälften miteinander flüssigkeitsdurchgängig verbinden. In einer Ausführungsform sind die Heizmittel und Fördermittel in den Gehäuseteilhälften selbst angeordnet. Diese Vorrichtungen können aber auch außerhalb der Gehäusehälften angeordnet sein.

In der erfindungsgemäßen Vorrichtung sind in den Gehäuseteilhälften bewegbare Folien vorgesehen, die die unter Druck stehende Flüssigkeit vom zu erwärmenden Behältnis trennen. Die membranartigen Folien, die das deformierbare Behältnis vollkommen und enganliegend umschließen, können den auf sie einwirkenden Druck vollkommen an den zu erwärmenden Beutel weitergeben. Damit ist auch ein guter Wärmeübergang zu dem erwärmenden Beutel gegeben.

Die Folien sind in einer bevorzugten Ausführungsform schon so geprägt, daß der zu erwärmende in seiner Größe und Form genormte deformierbare Beutel in eine Ausformung der Folie eingelegt werden kann. Ist der zu erwärmende Beutel eingelegt, so werden die Gehäuseteilhälften zugeklappt und die unter Druck stehende Flüssigkeit umströmt indirekt den zu erwärmenden Beutel.

In bevorzugter Ausführungsform können die Folien noch selbst beheizbar sein, indem elektrisch leitfähige Substanzen in der membranartigen Folie vorgesehen sind. Auch können Heizdrähte die membranartige Folie durchsetzen.

Das zu erwärmende deformierbare Behältnis in einem druckdichten Gefäß angeordnet. Von dem deformierbaren Behältnis läuft eine Leitung aus dem Gehäuse heraus, wobei die Leitung derart aus dem Gehäuse herausgeführt wird, daß die unter Druck stehende Flüssigkeit innerhalb des Gehäuses leckagefrei strömen kann. Im Gehäuse wirkt die unter Druck stehende Flüssigkeit, wie Wasser oder dergleichen, allseitig auf das deformierbare Behältnis. Die Flüssigkeit wirkt unter einem definierten Druck, der einstellbar ist, auf das Behältnis' und auch die Temperatur der Flüssigkeit ist einstellbar, so daß das Behältnis definiert gepreßt und erwärmt werden kann. Strömt die erwärmte Flüssigkeit in der erwähnten Art und Weise aus dem Gehäuse und/oder durch eine Leitung, die mit dem deformierbaren Behältnis verbunden ist, so können definierte flüssigkeitsmengen pro Zeiteinheit einer weiteren Erwärmungsvorrichtung unmittelbar zugeführt werden. Eine vollkommene Bilanzierung der im Beutel gespeicherten Flüssigkeit ist möglich, weil über die Leerpressung des Beutels keine Restmengen im Beutel zurückbleiben. Unterschiedliche Drücke der Flüssigkeit sind je nach Bedarf bis z. B. 500 mbar einstellbar.

An der aus dem Gehäuse herausführenden Leitung kann auch über eine von Hand oder elektrisch verschließbare Klemme vorgesehen sein. Ist der Verschließmechanismus der Klemme mit der Wärmezufuhr und/oder der Druckbeaufschlagung auf den im Gehäuse befindlichen Beutel gekoppelt und abgestimmt, so kann auch die schon den Aggregatzustand gewechselte Substanz weiter erwärmt werden. Es ist auch vorstellbar, daß die im Behältnis befindliche Substanz auf eine Temperatur erwärmt wird, die weit über dem Gefrierpunkt liegt. Ist die vorbestimmte Temperatur erreicht, so kann die Klemme geöffnet werden und die erwärmte flüssige Substanz kann unter Druck aus dem Gehäuse ausströmen. Je nachdem, wie stark das Behältnis druckbelastet wird, kann auch die Strömungsmenge pro Zeiteinheit oder ausströmender Flüssigkeit vorgegeben werden.

In einer Weiterbildung der medizinischen Auftauvorrichtung ist innerhalb des Gehäuses noch ein zusätzlicher Wärmeaustauscher vorgesehen, der unmittelbar an das Behältnis ankoppelbar ist. Das aus dem Behältnis strömende Medium kann im Wärmeaustauscher noch verstärkt erwärmt werden. Der Wärmeaustauscher selbst ist aus einem wärmeleitfähigen Material gefertigt, damit über eine kurze Weglänge eine effektive Erwärmung der den Wärmeaustauscher durchströmenden Flüssigkeit möglich ist.

Im Gehäuse wird die Zirkulation der Flüssigkeit durch eine im Gehäuse oder außerhalb des Gehäuses angeordnete Pumpe unterstützt und in den Gehäuseteilhälften können auch Propeller vorgesehen sein, die für eine besonders gute Strömung der unter Druck stehenden Flüssigkeit sorgen.

Weiterhin ist die erfindungsgemäße medizinische Auftauvorrichtung derart aufgebaut, daß mehrere Gehäuseteilhälften modulartig aneinander koppelbar sind, damit in der erfindungsgemäßen medizinischen Auftauvorrichtung auch mehrere Behältnisse gleichzeitig handhabbar sind. Die aus der medizinischen Auftauvorrichtung herausführenden Leitungssysteme sind miteinander verbindbar. Mit einer derartigen Vorrichtung können mehrere unterschiedliche Substanzen in unterschiedlichen Behältnissen gleichzeitig erwärmt, druckbeaufschlagt und dosiert und in einem bestimmten Mischungsverhältnis miteinander gemischt werden.

Die erfindungsgemäße medizinische Auftauvorrichtung zum Erwärmen und Leerpressen von deformierbaren Behältnissen entspricht damit allen erweiterten Anforderungen, die im medizinischen Anwendungsbereich an derartige Geräte gestellt werden. Die Gehäuseteilhälften sind einfach reinig- und sterilisierbar und damit keine Einmalartikel, sondern mehrfach verwendbar. Die im Handel erhältlichen Beutel, in denen beispielsweise das Blutplasma gespeichert ist, können mit einem einfachen Handgriff in die erfindungsgemäße medizinische Auftauvorrichtung eingelegt werden. Das Auftauen, Erwärmen und Leerpressen erfolgt in einem Gerät und nur bei besonderen Anwendungen sind weitere Geräte nachzuschalten, bevor die derart behandelte Lösung einem Patienten appliziert wird. Werden gefrorene Substanzen in der erfindungsgemäßen medizinischen Auftauvorrichtung erwärmt, so können die verflüssigten Anteile sofort aus dem Beutel gepreßt werden, und in der medizinischen Auftauvorrichtung werden dann nur noch die im Beutel verbleibenden gefrorenen Produktanteile erwärmt. Im medizinischen Anwendungsbereich sind die Drücke der auf das deformierbare Behältnis wirkenden Flüssigkeit stufenlos von z. B. 0 bis 500 mbar einstellbar und die Vorrichtung verfügt im Bedarfsfall über eine selbstregulierende Drucksteuerung. Die Flüssigkeit ist zusätzlich beheizbar und auf exakte Temperaturwerte einstellbar. Dabei ist es möglich, die Temperatur der strömenden Flüssigkeit zu erfassen und auszuwerten. Auf der Außenseite der medizinischen Auftauvorrichtung können der augenblickliche Druck der Flüssigkeit, die Temperatur und auch die Durchflußmenge der Flüssigkeit pro Zeiteinheit bzw. die in der medizinischen Auftauvorrichtung im Beutel befindliche medizinische Substanz angezeigt werden. Es ist denkbar, daß auch die Trübung der in dem Behältnis befindlichen Substanz geprüft und angezeigt wird. Mit dem erfindungsgemäßen Gerät ist es möglich, deformierbare Behältnisse unter unmittelbarer Druckeinwirkung leerzupressen und direkt oder indirekt über eine in den Geräteteilhälften angeordnete membranartige Folie zu beheizen. Die Folie gibt die in den Geräteteilhälften wirkenden Flüssigkeitsdrücke unmittelbar an das zwischen den Folien eingespannte Behältnis weiter.

Die medizinische Auftauvorrichtung kann mit mehreren Kammern ausgerüstet sein, d. h., daß mehrere deformierbare Behältnisse gleichzeitig in dieser medizinischen Auftauvorrichtung wärmebehandelt und leergepreßt werden können. Dabei ist es möglich, daß in den verschiedenen Gehäuseteilhälften unterschiedliche Drücke und unterschiedliche Temperaturen anstehen. Unterschiedliche Entleerungsgeschwindigkeiten der einzelnen deformierbaren Behältnissen sind möglich und Entleerungsprobleme, die aufgrund des unterschiedlichen Viskositätsverhaltens bekannt sind, treten bei einer erfindungsgemäßen Behandlung der deformierbaren Behältnisse nicht mehr auf. Weiterhin kann die gesamte, in den Behältnissen gespeicherte Substanz genutzt werden, weil das gesamte Behältnis aufgrund des anstehenden Flüssigkeitsdruckes vollkommen entleert werden kann.

Weitere Vorteile ergeben sich aus der Beschreibung und der beigefügten Zeichnung. Die erwähnten Ausführungsformen sind nicht als abschließende Aufzählung zu verstehen, sondern haben vielmehr beispielhaften Charakter. Die Erfindung ist in der Zeichnung dargestellt und wird anhand von Ausführungsbeispielen näher erläutert. Es zeigen:
- Fig. 1: Eine Draufsicht auf eine erfindungsgemäße medizinische Auftauvorrichtung, teilweise im Schnitt und mit freigelegter zweiter Gehäuseteilhälfte;
- Fig. 2: eine Ansicht in Pfeilrichtung II-II mit zusammengefügten Gehäuseteilhälften;
- Fig. 2a: einen Schnitt durch eine Ausführungsform mit strömungsfördernden Elementen und einer Heizung;
- Fig. 3: eine weitere Ausführungsform einer erfindungsgemäßen medizinischen Auftauvorrichtung in räumlicher Darstellung mit teilweise aufgeklappt gezeigten Gehäuseteilhälften, wobei die erfindungsgemäßen Folien der Übersichtlichkeit wegen nicht dargestellt sind.

Die einzelnen Figuren der Zeichnung zeigen den erfindungsgemäßen Gegenstand teilweise stark schematisiert und sind nicht maßstäblich zu verstehen. Die Gegenstände der einzelnen Figuren sind teilweise stark überproportional vergrößert dargestellt, damit ihr Aufbau besser gezeigt werden kann.

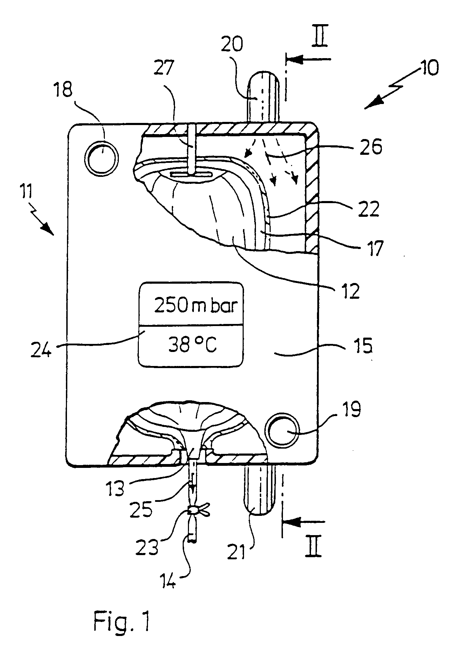

Fig. 1 zeigt einen Blutplasmaauftauer 10 mit einem Gehäuse 11, in dem ein schon unter geringer Druckeinwirkung deformierbarer Beutel 12 angeordnet ist. Der Beutel 12 weist ein Ansatzstück 13 auf, an das eine Leitung 14 ankoppelbar ist, über die die in dem Beutel 12 gespeicherte Substanz aus dem Gehäuse 11 ausfließen kann. Der Beutel 12 ist nicht nur bei Raumtemperatur deformierbar, sondern auch bei Temperaturen, die auf im Beutel gespeicherte Flüssigkeit abgestimmt sind.

Das Gehäuse 11 setzt sich aus einer ersten Gehäuseteilhälfte 15 und einer zweiten Gehäuseteilhälfte 16 zusammen. Der Beutel 12 ist zwischen den Gehäuseteilhälften 15, 16 in einem Freiraum 17 zwischen den Gehäuseteilhälften 15, 16 lagestabil gehalten.

In der Figur sind Anschlüsse 18 und 19 auf der Frontplatte des Gehäuses 11 eingezeichnet. Der Anschluß 18 dient für die erste Gehäuseteilhälfte 15 als Flüssigkeitseintrittstutzen und über den Anschluß 19 tritt die Flüssigkeit aus der Gehäuseteilhälfte 15 wieder aus. Über einen ersten Verbindungskanal 20 und einen zweiten Verbindungskanal 21 kann die erwärmte und unter Druck stehende Flüssigkeit von der ersten Gehäuseteilhälfte 15 in die zweite Gehäuseteilhälfte 16 strömen und von der zweiten Gehäuseteilhälfte 16 strömt die Flüssigkeit über den zweiten Verbindungskanal 21 wieder zurück in die erste Gehäuseteilhälfte 15, um danach über den Anschluß 19 aus dem Gehäuse 11 auszuströmen. Innerhalb der Gehäuseteilhälften 15, 16 können Strömungsleitsysteme angeordnet sein, die gewährleisten, daß die in das Gehäuse 11 einströmende Flüssigkeit gleichmäßig in den einzelnen Gehäuseteilhälften 15, 16 verteilt wird. Es versteht sich, daß die im Gehäuse 11 strömende Flüssigkeit so geführt wird, daß Kurzschlußströmungen auszuschließen sind.

Erfindungsgemäß sind die Gehäuseteilhälften 15, 16 zum Beutel 12 hin mit einer membranartigen Folie 22 ausgerüstet, die die einzelnen Gehäuseteilhälften 15, 16 vollkommen abschließt. Die Folien 22 können Ausprägungen aufweisen, in die die zu erwärmenden und auspreßbaren Beutel 12 einlegbar sind. Die Folie 22 selbst kann auch zusätzlich beheizbar ausgebildet sein, indem elektrisch leitfähige Substanzen, wie Carbonstoffe oder elektrische Leiter in die Folie eingeschweißt sind.

Soll ein in der medizinischen Auftauvorrichtung 10 zu behandelnder Beutel 12 erwärmt und leergepreßt werden, so ist es auch möglich an der Leitung 14 bzw. am Ansatzstück 13 eine Klemme 23 vorzusehen, über die das im Beutel 12 gespeicherte flüssige Produkt im Beutel 12 zurückgehalten werden kann. Der Beutel 12 kann je nach Bedarf über einen vorbestimmten Zeitraum wärmebehandelt und druckbehandelt werden. Sind die im Beutel 12 erwünschten Produkttemperaturwerte erreicht, so kann von Hand oder elektronisch die Klemme 23 geöffnet werden und je nach anstehendem Druck der Flüssigkeit in den Gehäuseteilhälften 15, 16 kann das im Beutel 12 gespeicherte Produkt mehr oder weniger schnell aus dem Beutel 12 herausgepreßt werden. Dabei kann auf der Vorderseite des Gehäuses 11 der anstehende Flüssigkeitsdruck angezeigt werden und auch die Temperatur der im Beutel 12 gespeicherten Substanz ist anzeigbar. Weiterhin kann im Display 24 die Ausströmmenge der Flüssigkeit pro Zeiteinheit und die im Gerät befindliche Substanz angezeigt werden. Wird die Klemme 23 geöffnet, so kann das erwärmte bzw. aufgetaute Produkt in Pfeilrichtung 25 aus dem Beutel 12 abströmen.

Mit gestrichelten Pfeilen 26 ist die Strömungsrichtung der aus der ersten Gehäuseteilhälfte 15 in die zweite Gehäuseteilhälfte 16 strömenden Flüssigkeit angedeutet. Mit 27 ist eine Aufhängevorrichtung, wie beispielsweise ein Band, angedeutet, über das der Beutel 12 zwischen den Gehäuseteilhälften 15, 16 lagestabil fixiert werden kann. Sind zwischen den Gehäuseteilhälften 15, 16 Folien 22 angeordnet, so halten diese Folien 22 den Beutel 12 lagestabil und eine zusätzliche Aufhängevorrichtung 27 ist nicht zwingend notwendig.

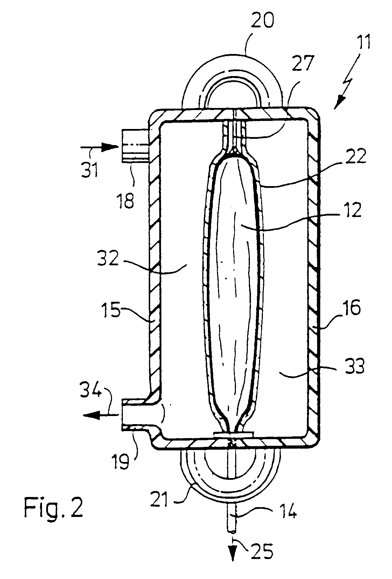

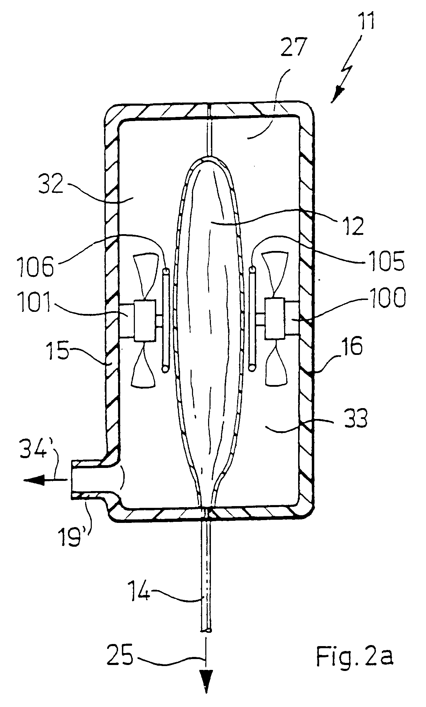

Fig. 2 zeigt eine Ansicht II-II im Schnitt. Die beiden Gehäuseteilhälften 15, 16 sind zusammengefügt. Die Gehäuseteilhälften sind zum Beutel 12 hin durch die Folien 22 abgeschlossen. Die Folien 22 liegen ganzflächig an dem Beutel 12 an. Der Beutel 12 ist zwischen den Gehäuseteilhälften 15, 16 noch über die Aufhängevorrichtung 27 gehalten, die an einer Gehäuseteilhälfte 15, 16 zusätzlich befestigt ist. Über die Anschlüsse 18, 19 kann erwärmte und unter Druck stehende Flüssigkeit den Gehäuseteilhälften 15, 16 zugeführt werden. In Pfeilrichtung 31 strömt beispielsweise die erwärmte und unter Druck stehende Flüssigkeit in die erste Gehäuseteilhälfte 15 ein. Von den Außenwandungen der ersten Gehäuseteilhälfte 15 und der Folie 22 ist ein erster Raum 32 begrenzt, in dem die unter Druck stehende und erwärmte Flüssigkeit ganzflächig auf die membranartige Folie 22 wirkt. Über die in der Figur gezeigten Verbindungskanäle 20, 21 kann aie über den Anschluß 18 einströmende Flüssigkeit auch in die zweite Gehäuseteilhälfte 16 strömen und von dieser aus strömt die Flüssigkeit zurück in die erste Gehäuseteilhälfte 15. In der zweiten Gehäuseteilhälfte 16 strömt die Flüssigkeit durch einen zweiten Raum 33, der durch die Außenwandungen der zweiten Gehäuseteilhälfte 16 und die Folie 22 begrenzt ist. In Pfeilrichtung 34 kann über den Anschluß 19 die im Gehäuse 11 befindliche Flüssigkeit ausströmen. Die aus dem Gehäuse 11 ausströmende Flüssigkeit kann im Kreislauf wiedererwärmt werden und erneut über den Stutzen 18 dem Gehäuse 11 zugeführt werden. Sind an der Gehäuseteilhälfte 16 vergleichbare Anschlüsse wie an der Gehäuseteilhälfte 15 vorgesehen, so kann die Flüssigkeit über die Anschlüsse 18, 19 einströmen und über Anschlüsse an der Gehäuseteilhälfte 16 abströmen.

Ist ein Beutel 12 in das Gehäuse 11 eingelegt und steht wie gezeigt die unter Druck stehende Flüssigkeit beidseitig in den Räumen 32, 33 an, so kann die in dem Beutel befindliche Substanz über die Leitung 14 in Pfeilrichtung 25 aus dem Beutel 12 herausgepreßt werden.

Fig. 2a zeigt einen Schnitt durch ein Ausführungsbeispiel mit nur einem Anschluß 19' für eine Flüssigkeitszufuhr in die Gehäuseteilhälfte 15, 16. Die Gehäuseteilhälften 15, 16 sind zusammengeklappt gezeigt. In dieser Stellung sind sie druckdicht aneinandergefügt. Der Beutel 12 ist über das Band 27 in den Gehäuseteilhälften 15, 16 lagestabil gehalten und über die Leitung 14 führt eine Verbindung aus den Gehäuseteilhälften 15, 16 heraus. Durch die Leitung 14 kann in Pfeilrichtung 25 eine im Beutel 12 gespeicherte Flüssigkeit bei Bedarf abfließen. In der jeweiligen Gehäuseteilhälfte 15, 16 ist ein Propeller 100, 101 angeordnet, die gewährleisten, daß eine in das Gehäuse 11 einströmende Flüssigkeit homogen und gleichmäßig innerhalb den Räumen 32, 33 verteilt wird. Die im Gehäuse 11 befindliche Flüssigkeit kann über Heizungen 105, 106 auf eine vorgegebene Temperatur erwärmt werden. Entgegen der Pfeilrichtung 34' kann eine Flüssigkeit den Räumen 32, 33 zugeführt werden. Über den Anschluß 19' kann der Flüssigkeitsdruck im Raum 32, 33 bedarfsabhängig auf- bzw. abgebaut werden.

Der deformierbare Beutel 12 ist von einer Flüssigkeit wie beispielsweise Wasser indirekt umströmt. Die Propeller 100 bzw. 101 beschleunigen die in den Räumen 32, 33 befindliche Flüssigkeit und über die Heizungen 105, 106 wird die Flüssigkeit erwärmt. Der gewünschte Druck wird über den Anschluß 19' in Pfeilrichtungen 34' auf- bzw. abgebaut. Die Räume 32, 33 sind wie bei anderen Ausführungsformen über Verbindungskanäle miteinander verbunden.

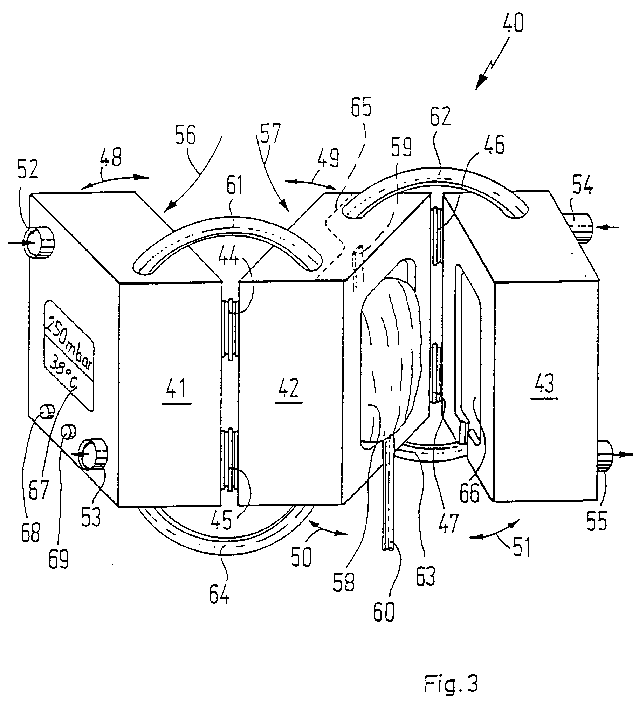

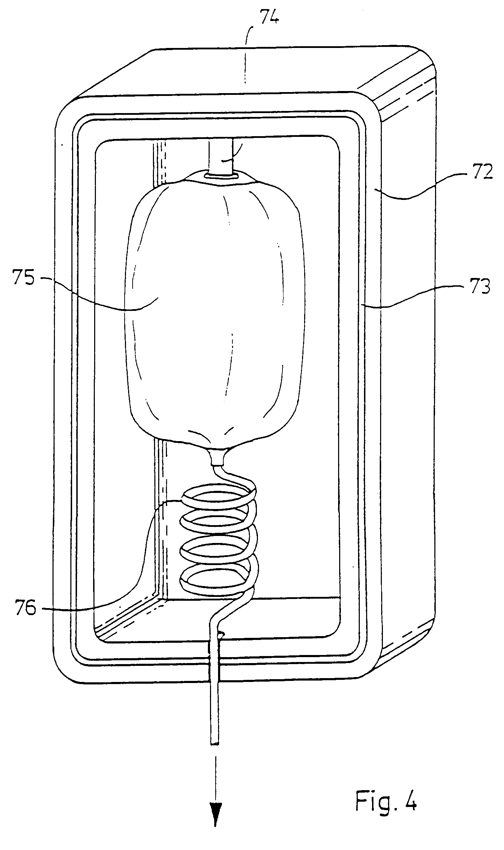

Fig. 3 zeigt eine weitere Ausführungsform einer medizinischen Auftauvorrichtung zum Erwärmen und Leerpressen von deformierbaren Behältnissen. Mit der in der Fig. 3 gezeigten Ausführungsform können gleichzeitig mehrere Behältnisse behandelt werden. Mit 40 ist eine medizinische Auftauvorrichtung gezeigt, die sich aus Gehäuseteilhälften 41, 42, 43 zusammensetzt. Die Gehäuseteilhälften sind über Scharniere 44, 45, 46, 47 verschwenkbar zusammengehalten. Die medizinische Auftauvorrichtung 40 ist modulartig aufgebaut und über die Scharniere können mehrere Gehäuseteilhälften aneinandergekoppelt werden. Über die Scharniere 44, 45, 46, 47 können auch elektrische Leitungen von der einen Gehäuseteilhälfte in die andere Gehäuseteilhälfte geführt werden. Dies ist dann notwendig, wenn in den einzelnen Gehäuseteilhälften 41, 42, 43 Temperaturen bzw. Flüssigkeitsdrücke gemessen, geregelt und gesteuert werden sollen.

Die Gehäuseteilhälften 41, 42, 43 können in Pfeilrichtungen 48, 49, 50, 51 zueinander verschwenkt werden. In der Figur sind die Gehäuseteilhälften teilweise geöffnet gezeigt. Die Gehäuseteilhälften 41, 42, 43 können vollkommen zusammengeklappt und verschlossen werden.

Die erwärmbare und unter Druck stehende Flüssigkeit, die in der medizinischen Auftauvorrichtung 40 auf die in die medizinische Auftauvorrichtung 40 eingelegten deformierbaren Behältnisse wirkt, wird der medizinischen Auftauvorrichtung 40 über den Anschluß 52 in Pfeilrichtung zugeführt. Über einen Anschluß 53 kann die Flüssigkeit wieder aus der medizinischen Auftauvorrichtung 40 abströmen. Auf der Gehäuseteilhälfte 43 sind auf der Außenseite ebenfalls Anschlüsse 54 und 55 vorgesehen, über die eine Flüssigkeit mit einer Temperatur und einem Druck, der unterschiedlich zu dem der Flüssigkeit ist, die über die Gehäuseteilhälfte 41 einströmt, in das Gehäuseteil 43 ein- und ausströmt.

Mit Pfeilen 56, 57 sind die Freiräume in den Gehäuseteilhälften 41, 42 angedeutet, in die das zu behandelnde Behältnis gelegt werden muß. Die Gehäuseteilhälfte 42 weist beidseitig Freiräume auf, in die ein Beutel 58 einlegbar ist. Der Beutel 58 ist über ein Band 59 der Gehäuseteilhälfte 42 gehalten und an dem Beutel 58 ist eine Leitung 60 angekoppelt, über die die im Beutel 58 gespeicherte Flüssigkeit aus dem Beutel 58 abströmen kann.

Werden Beutel 58 in die medizinische Auftauvorrichtung 40 eingelegt und werden die Gehäuseteilhälften zusammengeklappt d. h. verschlossen, so halten sie die eingelegten Beutel 58 eingeklemmt zwischen den Gehäuseteilhälften. Über die Anschlüsse 52 und 54 kann dann die unter Druck stehende und erwärmte Flüssigkeit in die medizinische Auftauvorrichtung 40 einströmen und über Verbindungskanäle 61, 62, 63, 64 verteilt sich die unter Druck stehende Flüssigkeit vollkommen gleichmäßig innerhalb der gezeigten Gehäuseteilhälften. In der Gehäuseteilhälfte 42 ist eine Zwischenwand 65 vorgesehen, die die über die Anschlüsse 52 und 54 einströmende Flüssigkeit voneinander trennt. Die Zwischenwand 65 ist druckstabil ausgebildet. Mit 66 ist ein Freiraum in der Gehäuseteilhälfte 43 gezeigt, in den der Beutel 58 hineinragt, wenn die Gehäuseteilhälften 42, 43 aufeinandergeklappt sind. Die Gehäuseteilhälften sind druckdicht zusammenfügbar, so daß die unter Druck stehende Flüssigkeit nicht über Öffnungsschlitze an der Vorrichtung unkontrolliert ausströmen kann. Die Flüssigkeit wirkt indirekt über nicht dargestellte Folien auf den Beutel 58.

Auf einer Außenseite der medizinischen Auftauvorrichtung 40 ist ein Display 67 angedeutet, über das alle wesentlichen Betriebsdaten der medizinischen Auftauvorrichtung anzeigbar und steuerbar sind. Mit 68, 69 sind Regel- und Steuerknöpfe angedeutet, über die die medizinische Auftauvorrichtung auch ein- und ausschaltbar ist. Über diese Regel- und Steuerknöpfe ist die Erwärmungstemperatur der einströmenden Flüssigkeiten über den Anschluß 52, 54 steuer- und regelbar und auch der Druck der einströmenden Flüssigkeiten 52 und 54 ist über diese Regel- und Steuerknöpfe vorgebbar. Die Flüssigkeit kann außerhalb und/oder innerhalb der medizinischen Auftauvorrichtung 40 erwärmt werden. Auch der Druck der Flüssigkeit kann innerhalb und außerhalb der medizinischen Auftauvorrichtung aufgebaut werden.

Die in den Figuren gezeigten medizinische Auftauvorrichtungen weisen Mittel auf, über die die Vorrichtungen beispielsweise an Infusionsständern befestigbar sind.

In einer zusammenklappbaren medizinischen Auftauvorrichtung, die aus Gehäuseteilhälften 15, 16 besteht, ist ein in den Gehäuseteilhälften 15, 16 gehaltener Beutel 12 wärme- und druckbehandelbar. Der Beutel 12 wird in der medizinischen Auftauvorrichtung 10 einer Wärmebehandlung derart ausgesetzt, daß das z. B. im Beutel gespeicherte tiefgefrorene Produkt aufgetaut wird und gleichzeitig, sobald es aufgetaut ist, aus dem Beute, 12 und aus dem Gehäuse 11 herausgepreßt werden kann. Dazu kann eine unter Druck stehende und erwärmte Flüssigkeit über einen Anschluß 18 in das Gehäuse 11 einströmen und über einen Anschluß 19 aus dem Gerät herausströmen. Die unter Druck stehende Flüssigkeit erwärmt im Gehäuse 11 die im Beutel 12 gespeicherte Substanz und preßt die flüssige Sustanz aus dem Beutel 12 und aus dem Gehäuse 11.

## Patentansprüche

1. Medizinische Auftauvorrichtung zum Auftauen von in deformierbaren Behältnissen gespeicherten medizinischen Produkten, insbesondere Blutplasma, Infusionen, Transfusionen oder dergleichen, mit einem Gehäuse (11), in dem das deformierbare Behältnis (12; 58) angeordnet ist, wobei das im Behältnis (12; 58) gespeicherte Produkt über eine Flüssigkeit einer Druckbehandlung aussetzbar ist, an das Behältnis (12; 58) eine Leitung (14; 60) angekoppelt ist, über die der im Behältnis (12; 58) gespeicherte, flüssige Produktanteil herausgepreßt wird, die medizinische Auftauvorrichtung eine erste Gehäuseteilhälfte (15) und eine zweite Gehäuseteilhälfte (16) aufweist, die zusammenfügbar sind, und wobei das Behältnis (12; 58) innerhalb eines von den Gehäuseteilhälften (15, 16) umschlossenen Freiraumes (17) angeordnet ist, dadurch gekennzeichnet, daß Heizmittel für die der Druckbehandlung dienende Flüssigkeit vorgesehen sind derart, daß das im Behältnis (12; 58) gespeicherte Produkt über die Flüssigkeit einer Wärmebehandlung aussetzbar ist, daß an den Gehäuseteilhälften (15, 16; 41, 42, 43) bewegbare, von der Flüssigkeit hinterströmte Folien (22) angeordnet sind, die die Gehäuseteilhälften (15, 16; 41, 42, 43) dicht abschließen, daß der Freiraum (17; 66) für die Aufnahme des Behältnisses (12; 58) zwischen den Folien (22) der Gehäuseteilhälften (15, 16; 41, 42, 43) bei zusammengefügten Gehäuseteilhälften (15, 16; 41, 42, 43) ausgebildet ist, wobei sich die Folien eng an das Behältnis (12; 58) anlegen, und daß an den Gehäuseteilhälften (15, 16; 41, 42, 43) Anschlüsse (18, 19; 52, 53, 54, 55) für die Führung der Flüssigkeit vorgesehen sind derart, daß das Behältnis (12; 58) vollkommen von der Flüssigkeit indirekt unströmt wird.

2. Vorrichtung nach Anspruch 1, dadurch gekennzeichnet, daß die Folien (22) beheizbar sind.

3. Vorrichtung nach Anspruch 1 oder 2, dadurch gekennzeichnet, daß die Flüssigkeit über in den Gehäuseteilhälften (15, 16; 41, 42, 43) anordenbaren Heiz- und/oder Fördermitteln erwärm- bzw. pumpbar ist.

4. Vorrichtung nach einem der Ansprüche 1 bis 3, dadurch gekennzeichnet, daß im Bereich eines Ansatzstückes (13) des Behältnisses (12) eine verschließbare Klemme (23) vorgesehen ist.

5. Vorrichtung nach einem der Ansprüche 1 bis 4, dadurch gekennzeichnet, daß im oder am Gehäuse (11) ein Wärmeaustauscher vorgesehen ist, durch den das aus dem Behältnis strömende Produkt erwärmbar ist.

6. Vorrichtung nach Anspruch 5, dadurch gekennzeichnet, daß der Wärmeaustauscher aus einem gut wärmeleitfähigen Material gefertigt ist.

7. Vorrichtung nach einem der Ansprüche 1 bis 6, dadurch gekennzeichnet, daß die Zirkulation der Flüssigkeit durch eine Pumpe und/oder einen Propeller bewirkbar ist, und daß die Pumpe und/oder der Propeller im oder außerhalb des Gehäuses (11) anordenbar sind.

8. Vorrichtung nach einem der Ansprüche 1 bis 7, dadurch gekennzeichnet, daß mehrere Gehäuseteilhälften (41, 42, 43) über Scharniere (44, 45, 46, 47) verschwenkbar zusammengehalten sind derart, daß mehrere Behältnisse (12; 58) gleichzeitig in der Vorrichtung (10; 40) handhabbar sind und daß Leitungssysteme der Behältnisse (12; 58) außerhalb der Vorrichtung miteinander verbindbar sind.

## Claims

1. Medical thawing device for the thawing of medical products stored in flexible containers, in particular blood plasma, infusions, transfusions or the like, comprising a housing (11) in which the flexible container (12; 58) is arranged, whereby the product stored in the container (12; 58) can be subjected to a pressure treatment by means of a fluid and a conduit (14; 60) can be coupled to the container (12; 58; 75) through which the liquid product portion stored in the container (12; 58) can be pressed out, the medical thawing device comprising a first housing section half (15) and a second housing section half (16) which can be put together and the container (12; 58) is arranged within a space (17) enclosed by the housing halves (15, 16), characterized in that heating means are provided for the pressure treatment fluid such that the product stored in the container (12; 58) can be subjected to a heat treatment by means of the fluid, and movable sheets (22) behind which the fluid flows are arranged on the housing halves (15, 16; 41, 42, 43) to seal the housing halves (15, 16; 41, 42, 43) and the space (17; 66) for the acceptance of the container (12; 58) is formed between the sheets (22) of the housing section halves (15, 16; 41, 42, 43) when the housing section halves (15, 16; 41, 42, 43) are placed together, whereby the sheets seat snuggly on the container (12; 58) and connection points (18, 19; 52, 53, 54, 55) are provided for on the housing section halves (15, 16; 41, 42, 43) for guiding the fluid such that the container (12; 58) is completely and indirectly surrounded by flowing fluid.

2. Device according to claim 1, characterized in that the sheets (22) can be heated.

3. Device according to claim 1 or 2, characterized in that the fluid can be warmed or pumped via heating and/or transport means which can be arranged in the housing halves (15, 16; 41, 42, 43; 72).

4. Device according to one of the claims 1 to 3, characterized in that a closable clamp (23) is provided in the vicinity of the container (12) adapter means (13).

5. Device according to one of the claims 1 to 4, characterized in that a heat exchanger is provided in or at the housing (11) for heating the product flowing out of the container.

6. Device according to claim 5, characterized in that the heat exchanger is manufactured from a good heat conducting material.

7. Device according to one of the claims 1 to 6, characterized in that the circulation of the fluid can be effected by means of a pump and/or a propeller and the pump and/or the propeller can be arranged within or outside of the housing (11).

8. Device according to one of the claims 1 to 7, characterized in that a plurality of housing section halves (41, 42, 43) are pivotably held together such that containers (12; 58) can be simultaneously handled by the device (10; 40) and container (12; 58) conduit systems can be connected to each other outside the device.

## Revendications

1. Dispositif médical de décongélation pour décongeler des produits médicaux, en particulier du plasma sanguin, des perfusions, des transfusions ou analogues, stockés dans des récipients déformables, comportant un boîtier (11) dans lequel le récipient déformable (12; 58) est disposé, dans le cas duquel le produit stocké dans' le récipient (12; 58) peut être exposé, au moyen d'un liquide, à un traitement sous pression, au récipient (12; 58) est raccordée une conduite (14; 60) au moyen de laquelle la portion liquide du produit stocké dans le récipient (12; 58) peut être extraite sous pression, le dispositif médical de décongélation présente une première moitié partielle (15) du boîtier une seconde moitié partielle (16) du boîtier qui peuvent être jointes, et dans le cas duquel le récipient (12; 58) est disposé à l'intérieur d'un espace libre (17) enclos par les moitiés partielles (15, 16) du boîtier, caractérisé par le fait que des éléments chauffants sont prévus, pour le liquide servant au traitement sous pression, de façon que le produit stocké dans le récipient (12; 58) puisse être exposé, au moyen du liquide, à un traitement thermique, que, sur les moitiés partielles (15, 16; 41, 42, 43) du boîtier sont disposées des feuilles mobiles (22) derrière lesquelles s'écoule le liquide et qui obturent de façon étanche les moitiés partielles (15, 5; 41, 42, 43) du boîtier, que l'espace libre (17; 66) est conçu pour recevoir le récipient (12; 58) entre les feuilles (22) des moitiés partielles (15, 6; 41, 42, 43) du boîtier lorsque les moitiés partielles (15, 16; 41, 42, 43) du boîtier sont jointes, les feuilles s'appliquant étroitement contre le récipient (12; 58), et qu'aux moitiés partielles (15, 16; 41, 42, 43) du boîtier, des raccords (18, 19; 52, 53, 54, 55) sont prévus pour guider le liquide de façon telle que le liquide s'écoule indirectement autour de la totalité du récipient (12; 58).

2. Dispositif selon la revendication 1, caractérisé par le fait que les feuilles (22) peuvent être chauffées.

3. Dispositif selon la revendication 1 ou 2, caractérisé par le fait que le liquide peut être chauffé ou pompé au moyen d'éléments chauffants et/ou de moyens de transport qui peuvent être disposés dans les moitiés partielles (15, 16; 41, 42 43) du boîtier.

4. Dispositif selon l'une des revendications 1 à 3, caractérisé par le fait que dans la zone d'un embout (13) du récipient (12) est prévue une pince (23).

5. Dispositif selon l'une des revendications 1 à 4, caractérisé par le fait que dans ou sur le boîtier (11) est prévu un échangeur de chaleur par l'intermédiaire duquel le produit qui s'écoute hors du récipient peut être chauffé.

6. Dispositif selon la revendication 5, caractérisé par le fait que l'échangeur de chaleur est fabriqué en un matériau bon conducteur de la chaleur.

7. Dispositif selon l'une des revendications 1 à 6, caractérisé par le fait que la circulation du liquide peut être opérée par une pompe et/ou par une hélice et que la pompe et /ou l'hélice peuvent être disposées dans le boîtier (11) ou à l'extérieur du boîtier.

8. Dispositif selon l'une des revendications 1 à 7, caractérisé par le fait que plusieurs moitiés partielles (41, 42, 43) de boîtier peuvent être maintenues ensemble, avec liberté de pivotement, par des charnières (44, 45, 46, 47) de façon telle que plusieurs récipients (12; 58) peuvent être simultanément traités dans le dispositif (10; 40) et que des systèmes de conduites des récipients (12; 58) peuvent être reliés l'un à l'autre à l'extérieur du dispositif.
